# FASCICULE DE BREVET EUROPEEN

(11) **EP 3 197 499 B1**
(45) Date de publication et mention de la délivrance du brevet: **15.11.2023**
(21) Numéro de dépôt: 15771186.2
(22) Date de dépôt: 07.09.2015
(51) Int. Cl.: A61K 47/34, A61K 47/32

(54) **ADMINISTRATION PAR VOIE ORALE D'AU MOINS UNE SUBSTANCE ACTIVE PHARMACEUTIQUE ET/OU ANTIGÉNIQUE**
ORALE VERABREICHUNG VON MINDESTENS EINEM PHARMAZEUTISCHEN UND/ODER ANTIGENEN WIRKSTOFF
ORAL ADMINISTRATION OF AT LEAST ONE PHARMACEUTICAL AND/OR ANTIGENIC ACTIVE SUBSTANCE

(30) Priorité: 23.09.2014 FR 1458966
(43) Date de publication de la demande: 02.08.2017
(73) Titulaire: Société d'Exploitation de Produits pour les Industries Chimiques SEPPIC, 75321 Paris cedex 07 (FR)
(72) Inventeur: BEN AROUS, Juliette, 92130 Issy Les Moulineaux (FR); DUPUIS, Laurent, 51100 Reims (FR); GAUCHERON, Jérôme, 81100 Castres (FR)
(74) Mandataire: Air Liquide
(86) Numéro de dépôt international: PCT/FR2015/052364
(87) Numéro de publication internationale: WO 2016/046464

(56) Documents cités:
- EP-A1- 1 726 600
- US-A1- 2001 028 884
- US-A1- 2004 198 904
- US-A1- 2010 233 196

## Description

La présente invention concerne un vecteur gastro-résistant destiné à être administré par voie orale et contenant au moins une substance active sur le plan thérapeutique et/ou prophylactique, son utilisation et son procédé de fabrication.

La voie orale présente de nombreux avantages pour la délivrance de médicaments ainsi que de vaccins. La biodisponibilité d'un principe actif administré par voie orale est très diminuée si le composé se trouve dégradé lors de son passage par les zones stomacales qui sont généralement riches en enzymes et se caractérisent par un pH très acide. Le mécanisme de digestion a en effet pour but de lyser un maximum de liaisons chimiques pour rendre absorbables les macromolécules, en les décomposant sous formes de plus petites molécules. Cela se traduit, pour les grosses molécules complexes que sont en général les principes actifs médicamenteux ou les vaccins (notamment ceux à base de protéines), par une perte d'efficacité.

Néanmoins, pour administrer des traitements par voie orale, deux stratégies majeures existent pour franchir la barrière gastrique :
- administrer de grosses quantités de principe actif : ainsi, même si une forte proportion est dégradée, il en restera une partie active ;
- véhiculer le principe actif dans un excipient qui le protégera de la dégradation en zone acide stomacale et le libérera en zone effective à pH neutre.

Cette seconde technologie s'appelle la mise en forme gastro-résistante. Elle est très utilisée sous forme de gélules (que l'on remplit avec le principe actif) ou de comprimés pelliculés avec au moins un film de polymère insoluble à pH acide et soluble à pH neutre.

Le remplissage des gélules ou le pelliculage des comprimés sont des procédés industriels très complexes, coûteux, et ne permettant pas de descendre à des unités d'administration de très petite taille (la taille limite étant celle des gélules et des comprimés). Pour le pelliculage, qui peut être envisagé pour des micro-granules de quelques millimètres de diamètre, cela nécessite des équipements et procédés de dépôt de films gastro-résistants très spécifiques, coûteux, nécessitant une ventilation à chaud pour évaporer le solvant utilisé pour déposer le film sur les granules supports en mouvement. Ce procédé n'est donc pas utilisable pour des principes actifs thermosensibles (comme par exemple des antigènes de vaccins) et les principes actifs dont les poussières sont toxiques. De plus, il est pratiquement impossible de part les contraintes de remplissage et de pelliculage, de travailler de manière aseptique pour produire des produits pharmaceutiques stériles.

Des formes liquides encapsulant des principes actifs hydrophiles dans des matrices huileuses (par exemple les émulsions eau-dans-huile) sont utilisées pour « protéger » et libérer progressivement des principes actifs et antigènes par la voie injectable (par exemple l'émulsion pour voie orale de l'exemple 2 du brevet EP0073006) mais ne résistent pas au passage du système digestif et sont dissociées et déstructurées lors du passage stomacal.

Des matrices de polymères épaississants assurant la formation d'un comprimé à délitement très lent assurent, par cette libération progressive, un effet gastro-résistant, comme décrit dans la demande de brevet WO2001078688. Si la cinétique de dissolution de la matrice est calquée sur les temps de transit estomac/intestin, la fraction terminale, qui se libère dans l'intestin, a franchi la barrière stomacale alors que la fraction initiale, dissoute dans l'estomac, a été digérée. On parle de matrice à effet retard plutôt que de véritable gastro-résistance. Dans ce cas, la matrice doit être de taille importante (par un exemple un comprimé à injecter par voie orale) à ajuster en fonction de la cinétique de transit. La cinétique de transit étant variable en fonction de l'espèce receveuse, le bolus alimentaire, l'âge, le type de digestion (poissons, ruminants, ...). Les performances de ce genre de matrices sont variables.

Les macromolécules de polymères, pour être utilisées dans des formulations pour la voie orale, doivent être solubles à pH neutre: en effet, la libération des principes actifs est ainsi assurée. Les molécules les plus grosses (les polymères de haut poids moléculaire) assurent la meilleure protection lors du passage dans les zones acides. Ces polymères ont cependant tous l'inconvénient de faire augmenter la viscosité des solutions aqueuses, les transformant en gel ou semi solides non versables, difficilement dosables ou absorbables.

Les polymères gastro-résistants ne peuvent être utilisés directement à forte teneur dans des solutions aqueuses car la forte viscosité qu'ils développent empêche l'écoulement. Par ailleurs, il est connu que les gels de ces polymères, à pH acide, forment un précipité dudit polymère.

Ces précipités, remis en pH neutre, permettent de libérer les molécules emprisonnées lors de la précipitation des polymères.

Les documents EP1 726 600 et US 2010/233196 décrivent une émulsion d'au moins une substance active comprenant une phase aqueuse (E) et une phase huileuse (H) sous forme d'émulsion de type eau dans huile (E/H) dans laquelle la phase aqueuse comprend au moins un principe actif et de 2% à 40% en poids de polymère hydrophile insoluble en phase aqueuse à pH < 6,5.

C'est pourquoi, les inventeurs de la présente invention ont cherché à mettre au point une solution permettant de résoudre les problèmes identifiés auparavant.

La présente invention a donc pour objet un vecteur gastro-résistant adapté pour l'administration par voie orale d'au moins une substance active pharmaceutique et/ou antigénique comprenant une phase aqueuse (E) et une phase huileuse (H) sous forme d'émulsion de type eau dans huile (E/H) dans laquelle la phase aqueuse comprend au moins un principe actif et de 2% à 40% en poids de polymère hydrophile insoluble en phase aqueuse à pH < 6,5, et dans laquelle la phase huileuse comprend au moins un tensioactif et au moins une huile constituée par de l'oléate d'éthyle.

L'invention a aussi pour objet :
Un vecteur tel que défini ci-dessus, caractérisé en ce que la phase huileuse comprend au moins un tensioactif et au moins une huile choisie parmi les esters d'acide gras, une huile minérale fluide, une huile végétale, le squalane.

Un vecteur tel que défini ci-dessus, caractérisé en ce que ledit polymère hydrophile est choisi parmi les polysaccharides constitués uniquement d'oses, les polysaccharides constitués de dérivés d'oses, les dérivés de cellulose et les polymères de type polyélectrolytes.

Un vecteur tel que défini ci-dessus, caractérisé en ce que le polymère hydrophile est choisi parmi l'hydroxypropylméthyle cellullose acéto succinate, l'hydroxypropylméthyle cellulose phatalte, le cellulose acétophtalate, la gomme laque, les copolymères de l'acide méthacrylique et de méthylméthacrylate, le diméthylaminoéthyl méthacrylate, méthacrylate de butyle.

Un vecteur tel que défini ci-dessus, caractérisé en ce que ledit polymère hydrophile est choisi parmi les dérivés de l'acrylamide, de l'acide acrylique et de la vinylpyrrolidone tels que les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone.

Un vecteur tel que défini ci-dessus, comprenant un adjuvant d'immunité choisi parmi le polyacrylate de sodium, les alginates, une microémulsion, des sels divalents.

Un vecteur tel que défini ci-dessus, caractérisé en ce que le principe actif est une substance susceptible d'être dénaturée ou dégradée lors d'une administration directe par voie orale et est choisi parmi un antigène, un médicament, un antiparasitaire, un antibiotique.

Vecteur tel que défini ci-dessus, caractérisé en ce que la quantité dudit polymère hydrophile ajoutée est supérieure à 4% en poids de la composition finale et inférieure à 20% en poids et de préférence comprise entre 10% en poids et 20% en poids de la composition finale.

Un véhicule gastro-résistant comprenant un ou plusieurs vecteurs tels que définis ci-dessus.

Une composition comprenant au moins un vecteur tel que défini précédemment ou un véhicule tel que défini ci-dessus.

L'utilisation d'un vecteur tel que défini précédemment ou un véhicule tel que défini ci-dessus pour obtenir un médicament actif par voie orale en thérapeutique humaine ou vétérinaire et possédant des propriétés curatives et/ou préventives et/ou permettant le diagnostic.

L'utilisation telle que définie ci-dessus pour la fabrication d'un produit pharmaceutique et/ou vétérinaire destiné à la vaccination par voie orale.

Un procédé de préparation d'un vecteur tel que défini précédemment comprenant les étapes suivantes :
a) préparation d'une phase huileuse comprenant une ou plusieurs huiles, et un système émulsionnant, comprenant un ou plusieurs tensioactifs émulsionnants et au moins un polymère hydrophile insoluble en phase aqueuse à pH < 6,5 et éventuellement de l'eau pour stabiliser le vecteur ;
b) ajout d'eau contenant le principe actif à rendre gastro-résistant sous agitation afin de former une émulsion de type eau dans huile (E/H) comprenant au moins 10% en poids d'eau.

Un procédé tel que défini ci-dessus caractérisé en ce que la phase aqueuse (E) de l'émulsion de type eau dans huile (E/H) contient un principe actif qui est une substance susceptible d'être dénaturée ou dégradée lors d'une administration directe par voie orale et est choisi parmi un antigène, un médicament, un antiparasitaire, un antibiotique.

Un procédé tel que défini ci-dessus, dans lequel ledit polymère est ajouté à l'étape a) sous forme de latex inverse huileux.

Un procédé tel que défini ci-dessus, dans lequel ledit polymère est ajouté à l'étape a) sous forme de poudre à disperser dans l'eau.

Un procédé tel que défini ci-dessus, dans lequel ledit polymère est ajouté à l'étape a) sous forme de dispersion aqueuse liquide ou de solution organique à émulsionner dans la composition huileuse de départ.

Un procédé tel que défini ci-dessus, dans lequel les étapes se déroulent à une température inférieure à 55°C et de préférence comprise entre 5°C et 35°C.

Une préparation pharmaceutique ou vétérinaire comprenant un vecteur préparé par le procédé défini précédemment.

En combinant la technologie de vecteur liquide huileux avec de fortes concentrations en polymère hydrophile gastro-résistant dans la phase aqueuse émulsionnée, les inventeurs de la présente invention ont réussi à formuler un excipient nouveau qui permet d'obtenir une émulsion fluide, versable, de type eau-dans-huile. En outre, les gouttes d'eau dispersées dans cette émulsion contiennent de fortes teneurs en polymères hydrophiles. Par ailleurs, les polymères hydrophiles gastro-résistants (insolubles et précipitant à pH acide mais soluble à pH neutre) n'épaississent pas l'émulsion puisqu'ils épaississent chaque goutte indépendamment.

De manière fort surprenante, il est possible d'incorporer des teneurs élevées en polymères hydrophiles, sans épaissir l'émulsion, par un procédé de mélange.

La préparation de l'émulsion gel dans huile gastro-résistante est effectuée comme suit :
1/ Un excipient huileux contenant une huile plus un système tensioactif est formulé pour donner, après addition d'environ 30% d'eau, une émulsion de type eau dans huile (E/H) fluide et stable (exemple d'huile : oléate éthyle, huile minérale fluide, huile végétale, squalane) (exemple de tensioactif : Montanide^{™} 888, Montanide^{™} 80, couple Montane^{™} 80 + Montanox^{™} 80).
2/ Dans cet excipient huileux, le polymère hydrophile (i.e. polymère hydrophile obtenu par la mise en oeuvre d'un procédé de polymérisation radicalaire en émulsion eau-dans-huile, comme par exemple les latex inverses commercialisés sous les noms de marque Simulgel^{™} et Sepigel par SEPPIC) ou de poudre (obtenus soit par la mise en oeuvre d'un procédé de polymérisation radicalaire par voie précipitante dans un solvant adapté, comme par exemple les polymères hydrophiles commercialisés sous le nom de marque Carbopol^{™} et Eudragit^{™}, soit par la mise en oeuvre d'un procédé de polymérisation radicalaire en émulsion eau-dans-huile suivi par une étape de précipitation du polymère hydrophile dans un solvant adapté ou par une étape d'atomisation) est dispersé sous forte agitation, ajouté sous forme, par exemple, de latex inverse huileux (exemple Simulgel^{™} et Sepigel^{™} commercialisé par SEPPIC) ou de poudre (exemple Carbopol^{™}, Eudragit^{™}) est dispersé sous forte agitation. La quantité de polymère ajoutée peut être élevée (2% à 20% en poids dans la formule finale), sans pour autant modifier la viscosité du mélange huileux.
3/ La phase aqueuse de l'émulsion, contenant l'actif (antigène, médicament, antiparasitaire, antibiotique) est ajoutée progressivement sous agitation. La formule est ainsi complétée.

Lors de l'ajout de la phase aqueuse contenant le principe actif, les gouttes forment avec les particules de polymère hydrophile dispersées, des particules gélifiées de petite taille (gouttes d'émulsion d'environ un micromètre de diamètre). Ces gouttes contiennent le principe actif à protéger emprisonné dans un agglomérat semi solide composé du polymère hydrophile hydraté.

Lors de l'utilisation de cette émulsion fluide pour une distribution par voie orale (par exemple, une cuillérée de ce liquide versée sur des aliments pour poulets ou pour poisson) l'émulsion va se déstructurer (les émulsions de type eau dans huile (E/H) sont détruites par les mécanismes de digestion). Des agglomérats de polymères vont ainsi être libérés et, en pH acide, s'insolubiliser, induisant une précipitation emprisonnant fortement le principe actif à véhiculer ce qui le protège du milieu extérieur destructeur. Le transit gastrique terminé, le passage en milieu neutre va permettre une solubilisation des polymères, se diluant dans les fluides intestinaux et libérant le principe actif. L'utilisation d'adjuvants d'immunité pour la vaccination par voie mucosale (polyacrylate de sodium, alginates, microémulsion, sels divalents) combinés à cette matrice gastro-résistante permet en outre d'augmenter la réponse immunitaire dans le cas de vaccination par voie orale.

Selon la présente invention, la phase grasse comprend une ou plusieurs huiles choisies notamment parmi :
- les huiles d'origine végétale, telles que l'huile d'amandes douces, l'huile de coprah, l'huile de monoï, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales et leurs esters méthyliques éthoxylés ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane et les huiles perfluorées. Les huiles de silicone sont également susceptibles d'être utilisées dans le cadre de la présente invention.

Parmi ces dernières, on peut plus particulièrement citer les diméthylpolysiloxanes, les méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles. Toutefois, pour des raisons pratiques, il peut être souhaitable que la phase grasse ne comprenne pas d'huile de silicone.

L'émulsion eau-dans-huile comprend généralement de 20% à 80% en poids, de préférence de 30% à 70% en poids, d'huile.

Parmi les tensioactifs émulsionnants susceptibles d'être utilisés dans le cadre de la présente invention, on citera notamment les N-acylés d'acides aminés et leurs sels ; les lipopeptides et leurs sels ; les esters de sorbitan comme par exemple le produit commercialisé sous la dénomination MONTANE^{™} 80 par la société SEPPIC ; les esters de polyglycérol comme par exemple les produits commercialisés sous la dénomination ISOLAN^{™} GI34 par BASF et PLUROL^{™} DIISOSTEARIQUE par GATTEFOSSE ; l'huile de ricin éthoxylée et l'huile de ricin hydrogénée éthoxylée comme par exemple le produit commercialisé sous la dénomination SIMULSOL^{™} 989 par la société SEPPIC ; le stéarate de glycérol ; les polyhydroxystéarates de polyglycol ou de polyglycérol comme par exemple les produits dénommés HYPERMER^{™} B246, ARLACEL^{™} P135 commercialisés par la société UNIQEMA, le produit dénommé DEHYMULS^{™} PGPH commercialisé par la société COGNIS, le produit dénommé DECAGLYN^{™} 5HS commercialisé par la société NIKKO ; les copolymères polyéthylèneglycol-alkylglycol comme le PEG-45 dodécylglycol copolymère tel que le produit commercialisé sous la dénomination ELFACOS ST 9^{™} par la société AKZO, les esters de sorbitan éthoxylés comme par exemple les produits commercialisés sous la dénomination MONTANOX^{™} par la société SEPPIC ; les acylats de protéines faiblement éthoxylés (de 1 à 3 groupements OE) ; la cire d'abeille éthoxylée comme par exemple le produit dénommé APIFIL^{®} commercialisé par la société GATTEFOSSE ; les émulsionnants cationiques comme les aminoxydes, le quaternium 82 et les tensio-actifs décrits dans la demande de brevet WO 96/00719 et principalement ceux dont la chaîne grasse comprend au moins 16 atomes de carbone ; les esters de sucrose, les esters de méthylglucoside ethoxylés ou non ; les acides gras éthoxylés ; les alcools gras éthoxylés ; les émulsionnants anioniques comme le décylphosphate ou le cétéarylsulfate ; le polyoxystéarate d'aluminium, tel que par exemple le produit commercialisé sous la dénomination MANALOX^{®} par la société RHODIA ; le stéarate de magnésium ; le stéarate d'aluminium.

Parmi les polymères de type polyélectrolytes, linéaires, ramifiés et/ou réticulés, qui conviennent tout particulièrement pour la mise en oeuvre du procédé de la présente invention, nous pouvons citer les dérivés de l'acrylamide, de l'acide acrylique et de la vinylpyrrolidone tels que les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone.

De tels polymères sont généralement préparés par un processus de polymérisation radicalaire en phase inverse, et sont notamment commercialisés respectivement sous les dénominations SIMULGEL^{™} EG, SEPIGEL^{™} 305, SIMULGEL^{™} NS, SIMULGEL^{™} 800 et SIMULGEL^{™} A par la société SEPPIC. Ils impliquent des formes partiellement ou totalement salifiées des monomères portant une fonction acide. Le ou les monomères correspondant(s) est (sont) dissous dans des gouttes d'eau dispersées dans une phase grasse à l'aide d'un système tensioactif émulsionnant. De tels polymères peuvent également être préparés par un processus de polymérisation radicalaire par voie précipitante ; le ou les monomères correspodant(s) est (sont) solubilisé(s) dans un solvant dans lequel le polymère formé est insoluble et précipite à l'issu de la réaction de polymérisation, laquelle est suivie d'une phase de filtration et de séchage de la poudre polymérique obtenue.

Avantageusement le poids sec dudit polyélectrolyte constitue entre 0,1% et 4% et de préférence entre 0,5% et 2% du poids de la phase aqueuse, si le procédé de préparation d'un tel polyélectrolyte résulte d'un processus de polymérisation précipitante ; ou le poids sec dudit polyélectrolyte constitue entre 0,25% et 4% et de préférence entre 0,5% et 2% du poids de la phase aqueuse si le procédé de préparation d'un tel polyélectrolyte résulte d'un processus de préparation de polymérisation en émulsion inverse.

Voici, ci-après, des exemples d'excipients huileux pour une émulsion de type eau dans huile.

Les adjuvants huileux sont de natures diverses.

Les adjuvants de Freund, les plus anciens, résultent de l'association d'une huile blanche minérale issue de la distillation de pétrole et d'un ester de mannitol contenant ou non une mycobactérie tuée.

Les adjuvants huileux commerciaux commercialisés sous le nom MONTANIDE^{™} ISA ont pour avantage de produire des émulsions de type eau dans huile (E/H), dites fluides, de viscosités de l'ordre de 500 mPa.s mesurée à l'aide d'un viscosimètre BROOKFIELD LVT équipé d'un mobile N°2 tournant à une vitesse de 60 tours par minute.

La demande internationale de brevet publiée sous le numéro WO 99/20305 divulgue l'utilisation de tensioactifs sur l'oléate de mannitol avec des huiles minérales comme le MARCOL^{™} 52 pour faire des émulsions, dites fluides.

En dehors des adjuvants huileux commerciaux prêts à l'emploi qui contiennent une ou plusieurs huiles et un ou plusieurs tensioactifs, il est possible d'utiliser des ester de sorbitol (Span^{™}) et des ester de sorbitol éthoxylés (Tween^{™}) par le fabricant de vaccin lui-même.

Ces tensioactifs en association avec une ou plusieurs huiles sont couramment utilisés pour fabriquer des compositions vaccinales émulsionnées de type eau-dans-huile E/H.

### EXEMPLE DE PREPARATION PAR AJOUT DE POLYMERE

La composition de l'adjuvant huileux gastro-résistant mise en oeuvre ici est un mélange optimisé d'un latex inverse de polyacrylate de sodium (PAS), de Montanide ISA et d'eau. Le latex inverse de PAS est la dispersion du polymère dans une matrice huileuse lors de la réaction de polymérisation radicalaire permettant de l'obtenir.

Exemple :
50% Latex inverse de PAS
20% Montanide^{™} ISA
30% Eau.

Une autre méthode posible peut être de doper une émulsion de type eau dans huile E/H spécifiquement formulée avec du polymère en poudre (par exemple un carbomère comme le carbopol^{™} 971 ou 940 ou autre grade) qui est dispersé sous forte agitation mécanique pour créer l'émulsion de base très chargée en polymère.

Toutes les structures chimiques de polymères insolubles à pH acide (< 6) dont la viscosité de leur gel aqueux chute d'au moins 50% et dont la solubilité diminue lorsque le pH passe de 7 à 2 peuvent être utilisées pour ce type de formulations.

Chimiquement, ces différentes familles d'épaississants peuvent être classées comme suit :
- Les polysaccharides constitués uniquement d'oses.
- Les polysaccharides constitués de dérivés d'oses.
- Les dérivés de cellulose.
- Les polymères de type polyélectrolytes, linéaires ou branchés et/ou réticulés.

On citera par exemple les polymères suivants :
- Sous forme de poudres (à redisperser dans l'eau de la formulation du vecteur huileux gastr-résistant (EHGR)) : Hydroxy Propyl Méthyle Cellulose Acéto Succinate- ex AQOAT^{™} de Shin Etsu, Hydroxypropyl Methyl Cellulose Phatalte - ex HP50^{™} et HP55^{™} de Shin Etsu, Cellulose acétophtalate, Gomme laque - ex Shellac^{™}, copolymères d'acide méthacrylique et de méthyle méthacrylate- ex Eudragit^{™} L 100 et Eudragit^{™} S100 de Evonik, PVAP - ex Sureteric de Colorcon, dimethylaminoethyl méthacrylate, butyle méthacrylate, et copolymères de méthyle méthacrylate - ex: Eudragit E100^{™} de Evonik.
- Sous forme de dispersion aqueuse liquide (à émulsionner dans l'EHGR : Ethylcellulose - ex AQUACOAT^{™} de FMC, Polyméthacrylate formulé avec PEG8000 - ex Acryl Eze^{™} de Colorcon, Polyméthacrylate en dispersion aqueuse à 30% - ex Eudragit L30D^{™}.
- Sous forme de dispersion ou solution organique (à émulsionner dans l'EHGR) : acide méthacrylique copolymères de méthyle méthacrylate dans l'alcool isopropylique- ex Eudragit^{™} S12.5 de Evonik.
- Ou sous forme de dispersion de polymère dans une émulsion eau dans huile (latex ou émulsion inverse) (à diluer cette fois dans l'EHGR), par exemple les gammes de produits Sepigel^{™} et simulgel^{™} de SEPPIC.

### DEMONSTRATION DES PROPRIETES DE GASTRO-RESISTANCE IN VITRO.

L'excipient (ou vecteur) huileux gastro-résistant (EHGR) de formule ci-dessous est émulsionné avec un mixeur à fort pouvoir dispersant (tel qu'un Silverson L4RT) en mélangeant 70% en poids de l'EHGR avec 30% en poids d'une solution de protéine modèle BSA.
50% Latex inverse de PAS.
20% Montanide^{™} ISA.
30% Eau.

Composition finale :
70% de vecteur huileux.
30% milieu aqueux contenant l'actif à rendre gastro résistant (par exemple : BSA).

### Composition finale de l'émulsion gastro résistante testée en pouvoir gastro-résistant

La concentration est de 4 mg de BSA par gramme d'émulsion de type eau dans huile (E/H) dans le mélange final.

Un test de libération est réalisé en deux temps au banc de dissolution ERWEKA avec 600g de milieu receveur et 60g d'émulsion sous agitation 100rpm (protocole extrait des recommandations pharmacopéales).

L'émulsion est placée, dans un premier temps, 24h dans une solution acide (HCl 0,1M) pendant 24 heures puis le pH de cette solution est remonté à un pH neutre et la solution est évaluée pendant 72 heures.

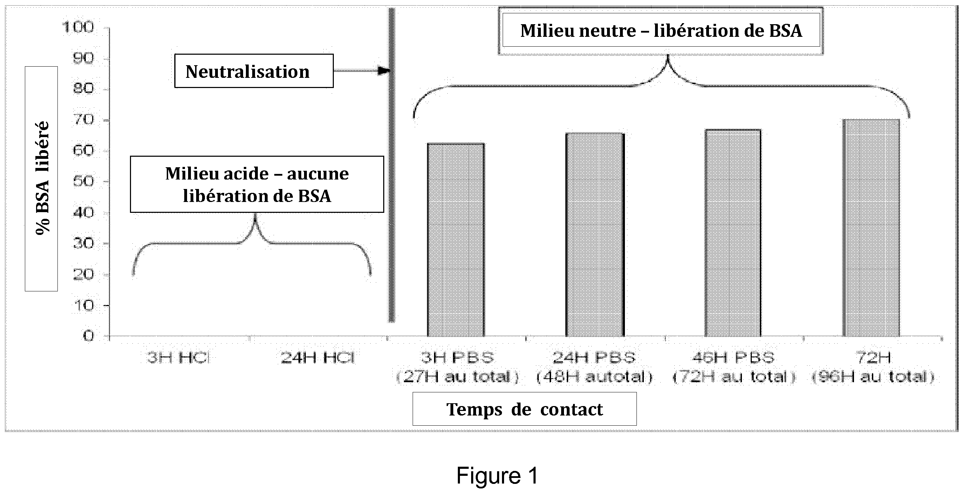

Les résultats de cette expérimentation sont illustrés à la figure 1.

En un jour en milieu acide (HCl 0.1M), il n'y a pas eu de libération de BSA. En trois jours en milieu neutre (PBS, pH=7,2), environ 75% de BSA ont été libérés.

Le milieu acide a pour but de mimer le passage stomacal et le milieu neutre, le passage intestinal.

Ces résultats démontrent le caractère gastro-résistant de la préparation : en effet, le principe actif encapsulé (ici la BSA) n'est pas libéré en milieu acide (proche de 0%) mais est libéré (environ 75%) lors de son passage en milieu neutre. La présence de BSA dosable indique que la protéine a été protégée contre la dégradation lors du passage en milieu acide.

Ces mêmes essais ont été conduits avec deux autres types de polymères dispersés dans l'EHGR :
- SEPIGEL^{™} 501 (copolymère, sous forme de latex inverse, réticulé d'acrylamide et d'acrylate de sodium, dispersé dans une huile minérale de paraffine).
- Eudragit^{™} L100-55 (polymère anionique possédant des groupements acide méthacryliques fonctionnels).

Les résultats sont présentés au tableau 1 en comparatif avec l'essai précédent effectué avec du polyacrylate de sodium.

**Tableau 1 : résultats in vitro de propriété de gastro-résistance mesurée sur des compositions d'EHGR obtenues à partir de différents polymères.**

| | Polyacrylate de sodium en dispersion (latex inverse) | SEPIGEL^{™} 501 (latex inverse) | Eudragit^{™} L100 (poudre) |
|---|---|---|---|
| BSA encapsulée dans EHGR | 4 mg / g d'EHGR | | |
| Fraction libérée après 24 heures dans HCl 0.1 M | 0 | 0 | 0 |
| Fraction libérée après 72 heures pH remonté à 7 | 75% | 86% | 82% |

Ces formulations présentent une forte gastro résistance pour des polymères de structures chimiques différentes et apportés dans la composition sous différentes formes (poudre et latex inverse).

### DEMONSTRATION DE PROPRIETES DE GASTRORESISTANCE IN VIVO

Trois modèles d'animaux ont été testés pour démontrer l'efficacité des vecteurs selon la présente invention dans des applications animales :
1/ Souris : des souris ont été gavées après anesthésie par 100 µl de cette préparation EHGR contenant 10 mg de protéine modèle (ovalbumine : OVA) en comparaison avec une solution aqueuse. Les réponses immunologiques mesurées dans les fèces se sont avérées très significativement supérieures pour le groupe ayant avalé l'EHGR par rapport au groupe ayant avalé la solution aqueuse. Cela indique donc que la protéine est bien arrivée intègre au système immunocompétent du système digestif situé après l'estomac (plaques de Peiers) sans être dégradée alors que l'ovalbumine en solution a été digérée dans l'estomac (cf. tableau 2).

**Tableau 2 : dosages des anticorps anti OVA dans les fèces de souris après gavage avec 10 mg d'OVA / souris.**

| Anticorps anti OVA (fèces) (UE/ml) | T0 | 30 JOURS (J) | 60 J | 90 J |
|---|---|---|---|---|
| Groupe témoin (pas ova) | 0 | 0 | 0 | 0 |
| OVA en solution aqueuse | 0 | 0 | 0 | 0 |
| OVA en EHGR | 0 | 100 | 200 | 200 |

Les valeurs de 100 et 200 obtenues lorsque l'excipient EHGR « vectorise » l'OVA sont très élevées et indiquent une réponse immunitaire forte. L'EHGR est donc bien adjuvant pour la vaccination par voie orale.

2/ Poissons : un antigène de Yersinia a été préparé dans l'EHGR en comparaison avec une solution aqueuse et versé sur des aliments en comprimés avant distribution aux poissons en élevage (truite arc en ciel). La protection induite (mesurée par épreuve virulente) a été nettement supérieure pour le groupe ayant avalé l'EHGR par rapport au groupe ayant avalé la solution aqueuse. Le groupe ayant avalé la solution aqueuse s'est comporté comme le groupe témoin négatif non vacciné (cf tableau 3).

**Tableau 3 : impact de l'excipient sur la protection induite par vaccination par voie orale avec l'antigène de Yersinia sur la truite arc en ciel.**

| | Vaccination J0 | Epreuve virulente J30 | Scores lésions J45 |
|---|---|---|---|
| Groupes témoin (pas d'antigène) | Pas d'antigène / 1000 poissons | ajout de bactéries dans le bain | +++ |
| Antigène en solution | 100 ml / 1000 poissons | ajout de bactéries dans le bain | +++ |
| Antigène en EHGR | 100 ml antigènes formulés en EHGR pour | ajout de bactéries dans le bain | Pas de lésions |
| | 1000 poissons | | |

L'utilisation de l'EHGR rend la vaccination par voie orale protectrice contre la maladie (pas de lésion) alors que les groupes témoins ne résistent pas à l'infection.

3/ Poules : de l'antigène contre la maladie de Newcastle a été administré par voie orale à des groupes de 20 poules âgées d'une semaine. Les vaccins, déposés dans l'eau de boisson, ont été formulés en EHGR ou en solution aqueuse. Les titres anticorps mesurés dans le sérum et la mortalité sont évalués dans cet essai.

L'antigène administré en solution aqueuse n'a pas apporté de production d'anticorps détectable et les animaux ont présenté les symptômes de la maladie (mortalité) similaire au groupe témoin n'ayant rien reçu.

Le groupe ayant reçu la préparation de l'antigène en EHGR présente des titres d'anticorps détectables et a été protégé contre l'infection lors de l'épreuve virulente (cf. tableau 4).

**Tableau 4 : résultats de protections induites par l'antigène de la maladie de Newcastle après administration par voie orale (eau de boisson) sous forme de vecteur gastro-résistant ou de solution aqueuse. Seul le groupe ayant reçu l'antigène vectorisé par l'EHGR est protégé contre la maladie et présente des titres d'anticorps.**

| | Vaccination J0 | Anticorps sériques | Survivants après épreuve virulente (%) |
|---|---|---|---|
| Groupes témoins (pas d'antigène) | Pas d'antigène | Non détectés | 7 |
| Antigène en solution | 50 ml d'antigène / 20 poulets | Non détectés | 5 |
| Antigène en EHGR | 50 ml d'antigène formulés en EHGR / 20 poulets | 4 log2 | 95 |

## Revendications

1. Vecteur gastro-résistant adapté pour l'administration par voie orale d'au moins une substance active pharmaceutique et/ou antigénique comprenant une phase aqueuse (E) et une phase huileuse (H) sous forme d'émulsion de type eau dans huile (E/H) dans laquelle la phase aqueuse comprend au moins un principe actif et de 2% à 40% en poids de polymère hydrophile insoluble en phase aqueuse à pH < 6,5, et dans laquelle la phase huileuse comprend au moins un tensioactif et au moins une huile constituée par de l'oléate d'éthyle.

2. Vecteur selon la revendication 1, **caractérisé en ce que** la phase huileuse comprend au moins un tensioactif et au moins une autre huile choisie parmi les esters d'acide gras, une huile minérale fluide, une huile végétale, le squalane.

3. Vecteur selon l'une des revendications précédentes **caractérisé en ce que** ledit polymère est choisi parmi les polysaccharides constitués uniquement d'oses, les polysaccharides constitués de dérivés d'oses, les dérivés de cellulose et les polymères de type polyélectrolytes.

4. Vecteur selon la revendication précédente **caractérisé en ce que** le polymère est choisi parmi l'hydroxypropylméthyle cellullose acéto succinate, l'hydroxypropylméthyle cellulose phatalte, le cellulose acétophtalate, la gomme laque, les copolymères de l'acide méthacrylique et de méthylméthacrylate, le diméthylaminoéthyl méthacrylate, méthacrylate de butyle.

5. Vecteur selon la revendication 3 **caractérisé en ce que** ledit polymère est choisi parmi les dérivés de l'acrylamide, de l'acide acrylique et de la vinylpyrrolidone tels que les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone.

6. Vecteur selon l'une des revendications précédentes comprenant un adjuvant d'immunité choisi parmi le polyacrylate de sodium, les alginates, une microémulsion, des sels divalents.

7. Vecteur selon l'une des revendications précédentes **caractérisé en ce que** le principe actif est une substance susceptible d'être dénaturée ou dégradée lors d'une administration directe par voie orale et est choisi parmi un antigène, un médicament, un antiparasitaire, un antibiotique.

8. Vecteur selon l'une des revendications précédentes **caractérisé en ce que** la quantité dudit polymère ajoutée est supérieure à 4% en poids de la composition finale et inférieure à 20% en poids et de préférence comprise entre 10% en poids et 20% en poids de la composition finale.

9. Vecteur selon l'une des revendications précédentes pour une utilisation comme véhicule gastro-résistant,

10. Médicament actif par voie orale en thérapeutique humaine ou vétérinaire et possédant des propriétés curatives et/ou préventives et/ou permettant le diagnostic comprenant un vecteur selon l'une des revendications 1 à 9.

11. Médicament selon la revendication 10, destiné à la vaccination par voie orale.

12. Procédé de préparation d'un vecteur tel que défini à l'une des revendications 1 à 8 comprenant les étapes suivantes :
a) préparation d'une phase huileuse comprenant une ou plusieurs huiles, et un système émulsionnant, comprenant un ou plusieurs tensioactifs émulsionnants et au moins un polymère hydrophile insoluble en phase aqueuse à pH < 6,5 et éventuellement de l'eau pour stabiliser le vecteur ;
b) ajout d'eau contenant le principe actif à rendre gastro-résistant sous agitation afin de former une émulsion de type eau dans huile (E/H) comprenant au moins 10% en poids d'eau.

13. Procédé selon la revendication précédente **caractérisé en ce que** la phase aqueuse (E) de l'émulsion de type eau dans huile (E/H) contient un principe actif qui est une substance susceptible d'être dénaturée ou dégradée lors d'une administration directe par voie orale et est choisi parmi un antigène, un médicament, un antiparasitaire, un antibiotique.

14. Procédé selon l'une quelconque des revendications 12 et 13, dans lequel le polymère est ajouté à l'étape a) sous forme de latex inverse huileux.

15. Procédé selon l'une quelconque des revendications 12 et 13, dans lequel le polymère est ajouté à l'étape a) sous forme de poudre à disperser dans l'eau.

16. Procédé selon l'une quelconque des revendications 12 et 13, dans lequel le polymère est ajouté à l'étape a) sous forme de dispersion aqueuse liquide ou de solution organique à émulsionner dans la composition huileuse de départ.

17. Procédé selon l'une quelconque des revendications 12 à 16 dans lequel les étapes se déroulent à une température inférieure à 55°C et de préférence comprise entre 5°C et 35°C.

## Patentansprüche

1. Magenresistenter Trägerstoff, der für die orale Verabreichung mindestens einer pharmazeutischen und/oder antigenartigen Wirksubstanz geeignet ist, wobei er eine wässrige Phase (W) und eine Ölphase (O) in Form einer Emulsion des Typs Wasser-in-Öl (W/O) umfasst, wobei die wässrige Phase mindestens einen Wirkstoff und 2 % bis 40 % nach Gewicht an hydrophilem Polymer umfasst, welches bei einem pH-Wert < 6,5 in der wässrigen Phase unlöslich ist, und wobei die Ölphase mindestens ein Tensid sowie mindestens ein Öl umfasst, welches aus Ethyloleat besteht.

2. Trägerstoff nach Anspruch 1, **dadurch gekennzeichnet, dass** die Ölphase mindestens ein Tensid und mindestens ein weiteres Öl umfasst, welches aus den Fettsäureestern, einem fließfähigen Mineralöl, einem pflanzlichen Öl, aus Squalan ausgewählt ist.

3. Trägerstoff nach einem der vorhergehenden Ansprüche, **dadurch gekennzeichnet, dass** das Polymer aus den Polysacchariden, welche ausschließlich aus Ösen bestehen, den Polysacchariden, die aus Derivaten von Ösen bestehen, den Cellulosederivaten und den polyelektrolytartigen Polymeren ausgewählt sind.

4. Trägerstoff nach dem vorhergehenden Anspruch, **dadurch gekennzeichnet, dass** das Polymer aus Hydroxypropylmethylcellulose-Acetosuccinat, Hydroxypropylmethylcellulosephthalat, Cellulose-Acetophthalat, Schellack, Copolymeren von Methacrylsäure und Methylmethacrylat, Dimethylaminoethylmethacrylat, Butylmethacrylat ausgewählt ist.

5. Trägerstoff nach Anspruch 3, **dadurch gekennzeichnet, dass** das Polymer aus den Derivaten von Acrylamid, von Acrylsäure und von Vinylpyrrolidon wie etwa den Copolymeren von Acrylsäure und 2-Methyl-[(1-oxo-2-propenyl)amino]-1-propansulfonsäure (AMPS), den Copolymeren von Acrylamid und 2-Methyl-[(1-oxo-2-propenyl)amino]-1-propansulfonsäure, den Copolymeren von 2-Methyl-[(1-oxo-2-propenyl)amino]-1-propansulfonsäure und (2-Hydroxyethyl)acrylat, dem Homopolymer von 2-Methyl-[(1-oxo-2-propenyl)amino]-1-propansulfonsäure, dem Homopolymer von Acrylsäure, den Copolymeren von Acryloylethyltrimethylammoniumchlorid und Acrylamid, den Copolymeren von AMPS und Vinylpyrrolidon, den Copolymeren von Acrylsäure und Alkylacrylaten, deren Kohlenstoffkette zwischen zehn und dreißig Kohlenstoffatome umfasst, den Copolymeren von AMPS und den Alkylacrylaten, deren Kohlenstoffkette zwischen zehn und dreißig Kohlenstoffatome umfasst, ausgewählt ist.

6. Trägerstoff nach einem der vorhergehenden Ansprüche, wobei er ein Immunitätsadjuvans umfasst, das aus Natriumpolyacrylat, den Alginaten, einer Mikroemulsion, zweiwertigen Salzen ausgewählt ist.

7. Trägerstoff nach einem der vorhergehenden Ansprüche, **dadurch gekennzeichnet, dass** es sich bei dem Wirkstoff um eine Substanz handelt, die bei einer direkten Verabreichung auf oralen Weg Gefahr liefe, denaturiert oder abgebaut zu werden, wobei er weiterhin aus einem Antigen, einem Medikament, einem parasitenbekämpfenden Mittel, einem Antibiotikum ausgewählt ist.

8. Trägerstoff nach einem der vorhergehenden Ansprüche, **dadurch gekennzeichnet, dass** die Menge des zugesetzten Polymers mehr als 4 Gewichts-% der fertigen Zusammensetzung und weniger als 20 Gewichts-% ausmacht, wobei sie vorzugsweise im Bereich von 10 Gewichts-% bis 20 Gewichts-% der fertigen Zusammensetzung liegt.

9. Trägerstoff nach einem der vorhergehenden Ansprüche, für eine Verwendung als magenresistenter Trägerstoff.

10. Medikament, das auf oralem Wege eine therapeutische Wirkung beim Menschen oder beim Tier entfaltet und heilende und/oder vorbeugende Eigenschaften hat und/oder eine Diagnosestellung ermöglicht, wobei es einen Trägerstoff nach einem der Ansprüche 1 bis 9 umfasst.

11. Medikament nach Anspruch 10, wobei es zur Impfung auf oralem Wege bestimmt ist.

12. Verfahren zur Herstellung eines Trägerstoffs, wie er in einem der Ansprüche 1 bis 8 definiert ist, wobei es die folgenden Schritte umfasst:
a) Herstellen einer Ölphase, die ein oder mehrere Öle und ein emulgierendes System, welches ein oder mehrere emulgierende Tenside umfasst, und mindestens ein hydrophiles Polymer, welches bei einem pH-Wert von < 6,5 in der wässrigen Phase unlöslich ist, und möglicherweise Wasser zur Stabilisierung des Vektors umfasst;
b) Zusetzen von Wasser, das den Wirkstoff enthält, welcher magenresistent formuliert werden soll, unter Rühren, um eine Emulsion des Typs Wasser-in-Öl (W/O) zu bilden, die mindestens 10 Gewichts-% an Wasser umfasst.

13. Verfahren nach dem vorhergehenden Anspruch, **dadurch gekennzeichnet, dass** die wässrige Phase (W) der Emulsion des Typs (W/O) einen Wirkstoff enthält, bei dem es sich um eine Substanz handelt, die bei einer direkten Verabreichung auf oralen Weg Gefahr liefe, denaturiert oder abgebaut zu werden, wobei er weiterhin aus einem Antigen, einem Medikament, einem parasitenbekämpfenden Mittel, einem Antibiotikum ausgewählt ist.

14. Verfahren nach einem beliebigen der Ansprüche 12 und 13, wobei das Polymer im Schritt a) in Form eines ölbasierten Inverslatex zugesetzt wird.

15. Verfahren nach einem beliebigen der Ansprüche 12 und 13, wobei das Polymer im Schritt a) in Form eines im Wasser zu dispergierenden Pulvers zugesetzt wird.

16. Verfahren nach einem beliebigen der Ansprüche 12 und 13, wobei das Polymer im Schritt a) in Form einer flüssigen wässrigen Dispersion oder einer organischen Lösung zugesetzt wird, welche dazu bestimmt ist, in der ölbasierten Ausgangszusammensetzung emulgiert zu werden.

17. Verfahren nach einem beliebigen der Ansprüche 12 bis 16, wobei die Schritte bei einer Temperatur erfolgen, die weniger als 55 °C beträgt und vorzugsweise im Bereich von 5 °C bis 35 °C liegt.

## Claims

1. Gastro-resistant vector suitable for oral administration of at least one pharmaceutical and/or antigenic active substance comprising an aqueous phase (W) and an oily phase (O) in the form of an emulsion of water-in-oil (W/O) type in which the aqueous phase comprises at least one active ingredient and from 2% to 40% by weight of hydrophilic polymer that is insoluble in an aqueous phase at pH < 6.5, and in which the oily phase comprises at least one surfactant and at least one oil consisting of ethyl oleate.

2. Vector according to Claim 1, **characterized in that** the oily phase comprises at least one surfactant and at least one other oil chosen from fatty acid esters, a fluid mineral oil, a vegetable oil, and squalane.

3. Vector according to either of the preceding claims, **characterized in that** said polymer is chosen from polysaccharides consisting only of monosaccharides, polysaccharides consisting of monosaccharide derivatives, cellulose derivatives and polymers of polyelectrolyte type.

4. Vector according to the preceding claim, **characterized in that** the polymer is chosen from hydroxypropylmethylcellulose acetate succinate, hydroxypropylmethylcellulose phthalate, cellulose acetate phthalate, shellac, copolymers of methacrylic acid and of methyl methacrylate, dimethylaminoethyl methacrylate, and butyl methacrylate.

5. Vector according to Claim 3, **characterized in that** said polymer is chosen from derivatives of acrylamide, of acrylic acid and of vinylpyrrolidone, such as copolymers of acrylic acid and of 2-methyl-[(1-oxo-2-propenyl)amino]-1-propanesulfonic acid (AMPS), copolymers of acrylamide and of 2-methyl-[(1-oxo-2-propenyl)amino]-1-propanesulfonic acid, copolymers of 2-methyl-[(1-oxo-2-propenyl)amino]-1-propanesulfonic acid and of (2-hydroxyethyl) acrylate, the homopolymer of 2-methyl-[(1-oxo-2-propenyl)amino]-1-propanesulfonic acid, the homopolymer of acrylic acid, copolymers of acryloylethyltrimethylammonium chloride and of acrylamide, copolymers of AMPS and of vinylpyrrolidone, copolymers of acrylic acid and of alkyl acrylates of which the carbon-based chain comprises between ten and thirty carbon atoms, and copolymers of AMPS and of alkyl acrylates of which the carbon-based chain comprises between ten and thirty carbon atoms.

6. Vector according to one of the preceding claims, comprising an immunity adjuvant chosen from sodium polyacrylate, alginates, a microemulsion, and divalent salts.

7. Vector according to one of the preceding claims, **characterized in that** the active ingredient is a substance capable of being denatured or degraded during direct oral administration and is chosen from an antigen, a medicament, an antiparasitic and an antibiotic.

8. Vector according to one of the preceding claims, **characterized in that** the amount of said polymer added is greater than 4% by weight of the final composition and less than 20% by weight and preferably between 10% by weight and 20% by weight of the final composition.

9. Vector according to one of the preceding claims, for use as a gastro-resistant carrier.

10. Medicament that is active via oral administration as a human or veterinary therapeutic and having curative and/or preventive properties and/or allowing diagnosis, comprising a vector according to one of Claims 1 to 9.

11. Medicament according to Claim 10, intended for oral vaccination.

12. Process for preparing a vector as defined in one of Claims 1 to 8, comprising the following steps:
a) preparing an oily phase comprising one or more oils, and an emulsifying system, comprising one or more emulsifying surfactants and at least one hydrophilic polymer that is insoluble in an aqueous phase at pH < 6.5 and optionally water for stabilising the vector;
b) adding water containing the active ingredient to be made gastro-resistant, with stirring, in order to form an emulsion of water-in-oil (W/O) type comprising at least 10% by weight of water.

13. Process according to the preceding claim, **characterized in that** the aqueous phase (W) of the emulsion of water-in-oil (W/O) type contains an active ingredient which is a substance capable of being denatured or degraded during direct oral administration and is chosen from an antigen, a medicament, an antiparasitic and an antibiotic.

14. Process according to either one of Claims 12 and 13, in which the polymer is added in step a) in the form of oily reverse latex.

15. Process according to either one of Claims 12 and 13, in which the polymer is added in step a) in the form of powder to be dispersed in the water.

16. Process according to either one of Claims 12 and 13, in which the polymer is added in step a) in the form of a liquid aqueous dispersion or of an organic solution to be emulsified in the starting oily composition.

17. Process according to any one of Claims 12 to 16, in which the steps take place at a temperature below 55°C and preferably between 5°C and 35°C.
